(19) Europäisches Patentamt
European Patent Office
Office européen des brevets

(11) EP 0 852 233 A1

(12) **EUROPEAN PATENT APPLICATION**
published in accordance with Art. 158(3) EPC

(43) Date of publication:
**08.07.1998 Bulletin 1998/28**

(21) Application number: **95932203.3**

(22) Date of filing: **21.09.1995**

(51) Int. Cl.$^6$: **C07K 7/64**, A61K 38/13

(86) International application number:
**PCT/JP95/01906**

(87) International publication number:
**WO 97/11092 (27.03.1997 Gazette 1997/14)**

(84) Designated Contracting States:
**DE FR GB IT**

(71) Applicant:
**NIPPON SHINYAKU COMPANY, LIMITED**
**Minami-ku Kyoto-shi Kyoto 601 (JP)**

(72) Inventors:
- **KONNO, Kiyotaka**
  **Kyoto-shi, Kyoto 610-1146 (JP)**
- **SETO, Takashi**
  **Kyoto-shi, Kyoto 612-8469 (JP)**
- **YOSHIFUSA, Hiroto**
  **Takatsuki-shi, Osaka 569-1025 (JP)**
- **EZURE, Yoji**
  **Otsu-shi, Shiga 520-2133 (JP)**

(74) Representative:
**Strych, Werner Maximilian Josef, Dr. et al**
**Hansmann & Vogeser,**
**Patent- und Rechtsanwälte,**
**Albert-Rosshaupter-Strasse 65**
**81369 München (DE)**

# (54) CYCLOSPORIN PHOSPHATE DERIVATIVES AND MEDICINAL COMPOSITION

(57) The invention provides a novel cyclosporin compound which is water-soluble, improved in the amount of absorption and absorption rate after oral administration, with low nephrotoxicity, and a pharmaceutical composition comprising the same compound.

The compound of the invention includes such cyclosporin phosphate derivatives as [phosphonothreonine$^2$]cyclosporin A, [phosphono-4-hydroxynorvaline$^2$]cyclosporin A, [phosphono-D-serine$^8$]cyclosporin A, [phosphonothreonine$^2$, phosphono-D-serine$^8$]cyclosporin A, etc. or salts thereof, and solvates thereof.

EP 0 852 233 A1

## Description

### TECHNICAL FIELD

The present invention relates to cyclosporin phosphate derivatives. More particularly, the invention relates to a medicine and, in particular, to a cyclosporin phosphate derivative which is useful as an immunosuppressant drug.

### BACKGROUND ART

Cyclosporins are 11-amino acid residue-cyclic peptides produced by Tolypocladium inflatum and other fungi imperfecti. Many cyclosporins act on lymphocytes, specifically and reversibly, to express potent immunosuppressant and antiinflammatory actions. These pharmacological activities are well documented (e.g. R. Y. Calne, Trend in Pharmaceutical Sciences: Immunosupressive in Clinical Organ Grafting, Vol. 1 (1979), 21-22; R. Y. Calne, Nephron, 26 (1980), 57-63).

Cyclosporins are compounds listed in Merck Index, among other reference books, and many specific compounds are already known. R. M. Wenger et al. [Process in the Chemistry of Organic Natural Products, Vol. 50, 157, Springer-Verlag, edited by W. Herz et al.], for instance, present a list of cyclosporins produced by fungi. The following is the list.

| | 1 | 2 | 3 | 4 | 5 | 6 | 7 | 8 | 9 | 10 | 11 |
|---|---|---|---|---|---|---|---|---|---|---|---|
| A | MeBmt | L-Abu | Sar | L-MeLeu | L-Val | L-MeLeu | L-Ala | D-Ala | L-MeLeu | L-MeLeu | L-MeVal |
| B | MeBmt | L-Ala | Sar | L-MeLeu | L-Val | L-MeLeu | L-Ala | D-Ala | L-MeLeu | L-MeLeu | L-MeVal |
| C | MeBmt | L-Thr | Sar | L-MeLeu | L-Val | L-MeLeu | L-Ala | D-Ala | L-MeLeu | L-MeLeu | L-MeVal |
| D | MeBmt | L-Val | Sar | L-MeLeu | L-Val | L-MeLeu | L-Ala | D-Ala | L-MeLeu | L-MeLeu | L-MeVal |
| E | MeBmt | L-Abu | Sar | L-MeLeu | L-Val | L-MeLeu | L-Ala | D-Ala | L-MeLeu | L-MeLeu | L-Val |
| F | deoxyMeBmt | L-Abu | Sar | L-MeLeu | L-Val | L-MeLeu | L-Ala | D-Ala | L-MeLeu | L-MeLeu | L-MeVal |
| G | MeBmt | L-Nva | Sar | L-MeLeu | L-Val | L-MeLeu | L-Ala | D-Ala | L-MeLeu | L-MeLeu | L-MeVal |
| H | MeBmt | L-Abu | Sar | L-MeLeu | L-Val | L-MeLeu | L-Ala | D-Ala | L-MeLeu | L-MeLeu | D-MeVal |
| I | MeBmt | L-Val | Sar | L-MeLeu | L-Val | L-MeLeu | L-Ala | D-Ala | L-MeLeu | L-Leu | L-MeVal |
| K | deoxyMeBmt | L-Val | Sar | L-MeLeu | L-Val | L-MeLeu | L-Ala | D-Ala | L-MeLeu | L-MeLeu | L-MeVal |
| L | Bmt | L-Abu | Sar | L-MeLeu | L-Val | L-MeLeu | L-Ala | D-Ala | L-MeLeu | L-MeLeu | L-MeVal |
| M | MeBmt | L-Nva | Sar | L-MeLeu | L-Nva | L-MeLeu | L-Ala | D-Ala | L-MeLeu | L-MeLeu | L-MeVal |
| N | MeBmt | L-Nva | Sar | L-MeLeu | L-Val | L-MeLeu | L-Ala | D-Ala | L-MeLeu | L-Leu | L-MeVal |
| O | L-MeLeu | L-Nva | Sar | L-MeLeu | L-Val | L-MeLeu | L-Ala | D-Ala | L-MeLeu | L-MeLeu | L-MeVal |
| P | Bmt | L-Thr | Sar | L-MeLeu | L-Val | L-MeLeu | L-Ala | D-Ala | L-MeLeu | L-MeLeu | L-MeVal |
| Q | MeBmt | L-Abu | Sar | L-Val | L-Val | L-MeLeu | L-Ala | D-Ala | L-MeLeu | L-MeLeu | L-MeVal |
| R | MeBmt | L-Abu | Sar | L-Leu | L-Val | L-Leu | L-Ala | D-Ala | L-MeLeu | L-MeLeu | L-MeVal |
| S | MeBmt | L-Thr | Sar | L-Val | L-Val | L-MeLeu | L-Ala | D-Ala | L-MeLeu | L-MeLeu | L-MeVal |
| T | MeBmt | L-Abu | Sar | L-MeLeu | L-Val | L-MeLeu | L-Ala | D-Ala | L-MeLeu | L-Leu | L-MeVal |
| U | MeBmt | L-Abu | Sar | L-MeLeu | L-Val | L-Leu | L-Ala | D-Ala | L-MeLeu | L-MeLeu | L-MeVal |
| V | MeBmt | L-Abu | Sar | L-MeLeu | L-Val | L-MeLeu | L-Abu | D-Ala | L-MeLeu | L-MeLeu | L-MeVal |
| W | MeBmt | L-Thr | Sar | L-MeLeu | L-Val | L-MeLeu | L-Ala | D-Ala | L-MeLeu | L-MeLeu | L-Val |
| X | MeBmt | L-Nva | Sar | L-MeLeu | L-Val | L-MeLeu | L-Ala | D-Ala | L-Leu | L-MeLeu | L-MeVal |
| Y | MeBmt | L-Nva | Sar | L-MeLeu | L-Val | L-Leu | L-Ala | D-Ala | L-MeLeu | L-MeLeu | L-MeVal |
| Z | * | L-Abu | Sar | L-MeLeu | L-Val | L-MeLeu | L-Ala | D-Ala | L-MeLeu | L-MeLeu | L-MeVal |

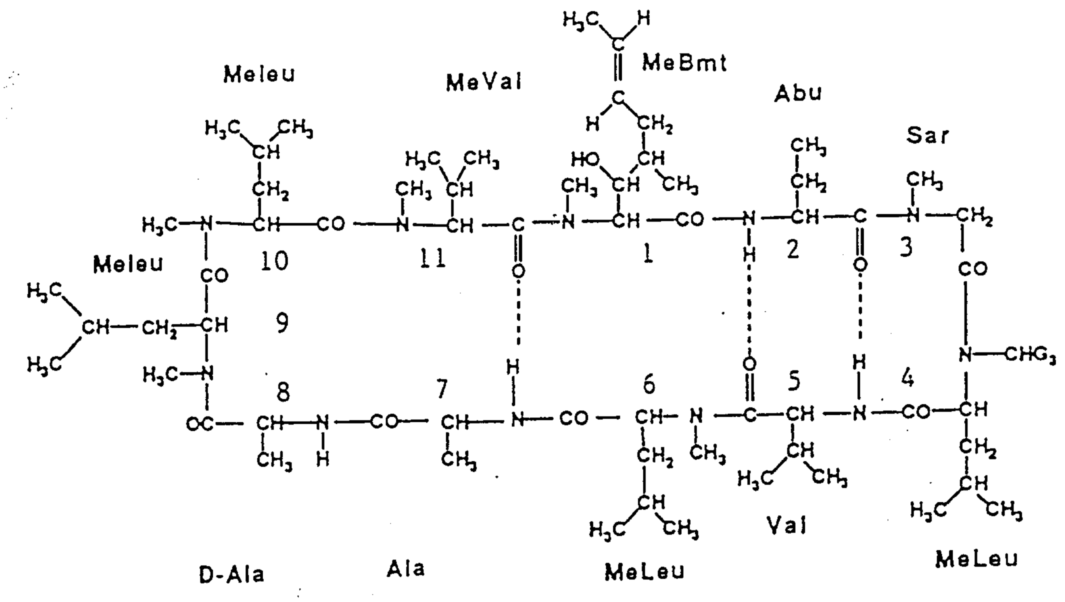

Referring to the table, the numerals of 1 to 11 in the top horizontal row are the position numbers of amino acids corresponding to those used in the chemical formula of cyclosporin A and the alphabet letters of A to Z in the vertical row at left represent species of cyclosporin. MeBmt means N-methyl-(4R)-4-but-2E-en-1-yl-4-methyl-(L) threonine of chemical formula [II] or the corresponding dihydro compound of chemical formula [III]; DeoxyBmt means N-methyl-(4R)-4-but-2E-en-1-yl-4-methyl-(L)-α-aminobutyric acid; Bmt means (4R)-4-but-2E-en-1-yl-4-methyl-(L)-threonine; * means N-methyl-2-aminooctanoic acid; Sar means sarcosine (N-methyl-L-glycine); Abu means L-α-aminobutyric acid; Ala means alanine; Leu means leucine; MeLeu means N-methyl-L-leucine; MeVal means N-methyl-L-valine; Nva means norvaline; Thr means threonine; Val means valine; the prefix D means the D-form; and the prefix L means the L-form.

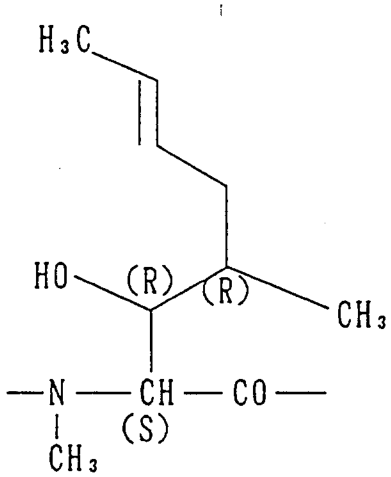

〔II〕

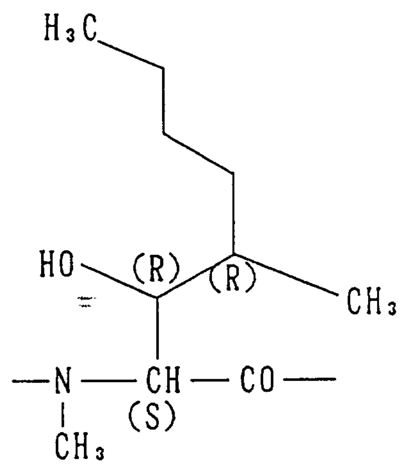

〔III〕

Cyclosporins as substituted by D-serine for the residue in the 8-position are also known (R. Traber et al., J. Antibiot., 42, 591-597, 1989). Cyclosporins as substituted by 3-hydroxynorvaline or 4-hydroxynorvaline for the residue in the 2-position have also been reported (C. Papageogiou et al., Bioorganic & Medical Chemistry Letters, 13, 2529-2564, 1993).

Other cyclosporin compounds are also disclosed in Japanese Kokai Tokkyo Koho S52-59180, Kokai Tokkyo Koho S56-128725, Kokai Tokkyo Koho S57-140753, Kokai Tokkyo Koho S60-215700, Kokai Tokkyo Koho S61-212599, Kokai Tokkyo Koho S62-77399, and Kokai Tokkyo Koho H2-137.

Paying attention to the immunosuppressant and other activities of cyclosporins, various pharmaceutical compositions containing them have so far been proposed (e.g. Japanese Kohyo Tokkyo Koho H5-500964, Kohyo Tokkyo Koho H5-503306, Kokai Tokkyo Koho H5-208996, Kokai Tokkyo Koho H5-310591, and Kohyo Tokkyo Koho H5-507903). Actually, cyclosporin A is commercially available in such dosage forms as capsules, elixirs, and injections.

However, cyclosporins so far known have the disadvantage that they are only sparingly soluble in water, nephrotoxic, and when administered orally, not well absorbed (on the side effects of cyclosporin A: M. J. Mihatsch et al., Toxicology Letters, 46, 125-139, 1989; B. Ryffel, Arch Toxicology, 53, 107-141, 1983).

Meanwhile, no information is available on the phosphate of a cyclosporin and, for that matter, a pharmaceutical composition containing such an ester. Moreover, there is no prior art case in which improvements in water solubility and absorption and reduction in nephrotoxicity were achieved by phosphorylating a polypeptide.

DISCLOSURE OF THE INVENTION

The present invention has for its object to provide a novel cyclosporin compound which is water-soluble, well absorbed on oral administration, and only sparingly nephrotoxic and a pharmaceutical composition comprising the same compound.

The present invention is directed to a cyclosporin phosphate derivative which is available upon total or partial phosphorylation [-P(O)(OH)$_2$] of hydroxyl groups of a cyclosporin having hydroxyl group-containing amino acids as its constituent amino acids or a salt thereof, or a solvate (inclusive of hydrate) thereof.

The present invention is characterized in that the hydroxyl groups of a cyclosporin having hydroxyl group-containing amino acids as its constituent amino acids have been totally or partly phosphorylated.

As the cyclosporin containing hydroxy-amino acid residues, a compound of the following chemical formula [I] or a salt thereof, or a solvate (inclusive of hydrate) thereof (hereinafter referred to collectively as the compound of the invention) can be mentioned.

$$\text{MeBmt} - A_1 - \text{Sar} - A_2 - A_3 - A_4 - A_5 - A_6 - A_7 - A_8 - A_9$$

1 2 3 4 5 6 7 8 9 10 11

[ I ]

wherein $A_1$ represents D- or L-α-aminobutyric acid, D- or L-alanine, D- or L-threonine, D- or L-valine, D- or L-3-hydroxynorvaline, or D- or L-4-hydroxynorvaline; $A_2$ represents D- or L-N-methylleucine, D- or L-leucine, or D- or L-valine; $A_3$ represents D- or L-valine or D- or L-norvaline; $A_4$ represents D- or L-leucine or D- or L-methylleucine; $A_5$ represents D- or L-alanine or D- or L- aminobutyric acid; $A_6$ represents D- or L-alanine or D- or L-serine; $A_7$ represents D- or L-methylleucine or D- or L-leucine; $A_8$ represents D- or L-methylleucine or D- or L-leucine, $A_9$ represents D- or L-valine or D- or L-methylvaline; MeBmt and Sar are as respectively defined hereinbefore; the numerals 1-11 represent the position numbers of amino acid residues.

Preferred are the compounds in which $A_1$ = L-threonine or $A_6$ = D-serine. Further preferred are [threonine$^2$]cyclosporin A, [4-hydroxynorvaline$^2$]cyclosporin A, [D-serine$^8$]cyclosporin A, and [threonine$^2$, D-serine$^8$]cyclosporin A.

Here, the nomenclature of cyclosporins as used in this specification is now explained.

(1) All amino acids, not specifically designated by the prefix D-, are L-amino acids.

(2) With cyclosporin A being taken as a standard, a cyclosporin containing any amino acid residue that is different from the corresponding residue of cyclosporin A is designated by indicating the particular different amino acid residue and its position as follows. For example, [threonine$^2$]cyclosporin A and [threonine$^2$, D-serine$^3$]cyclosporin A are cyclosporin A with threonine substituted for the amino acid in the 2-position and cyclosporin A substituted by threonine for the amino acid in the 2-position and D-serine for the amino acid in the 8-position, respectively. [Phosphonothreonine$^2$]cyclosporin A and [phosphono-D-serine$^8$]cyclosporin A are the compound corresponding to cyclosporin A substituted by L-threonine for the amino acid in the 2-position, with the hydroxyl group of the L-threonine having been phosphorylated, and the compound corresponding to cyclosporin A substituted by D-serine for the amino acid in the 8-position, with the hydroxyl group of the D-serine having been phosphorylated, respectively.

(The positions of amino acids correspond to those indicated in Chemical Formula [I]). [Phosphono-D-serine$^8$]cyclosporin A disodium salt is [phosphono-D-serine$^8$]cyclosporin A in which the phosphoric moiety has been converted to a disodium salt.

The following is a partial list of the compounds of the present invention.

[Phosphonothreonine$^2$]cyclosporin A,
[Phosphonothreonine$^2$]cyclosporin A monosodium salt,
[Phosphonothreonine$^2$]cyclosporin A disodium salt,
[Phosphonothreonine$^2$]cyclosporin A dipotassium salt,
[Phosphono-D-serine$^8$]cyclosporin A,
[Phosphono-D-serine$^8$]cyclosporin A monosodium salt,
[Phosphono-D-serine$^8$]cyclosporin A disodium salt,
[Phosphono-D-serine$^8$]cyclosporin A dipotassium salt,
[Phosphono-D-serine$^8$]cyclosporin A dibenzylamine salt,
[Phosphonothreonine$^2$, phosphono-D-serine$^8$]cyclosporin A,
[Phosphonothreonine$^2$, phosphono-D-serine$^8$]cyclosporin A disodium salt,
[Phosphonothreonine$^2$, phosphono-D-serine$^8$]cyclosporin A tetrasodium salt,
[Phosphonothreonine$^2$, phosphono-D-serine$^8$]cyclosporin A tetrapotassium salt,
[Phosphono-D-threonine$^8$]cyclosporin A,
[Phosphono-D-threonine$^8$]cyclosporin A disodium salt,
[Phosphono-D-threonine$^8$]cyclosporin A dipotassium salt,
[Phosphono-D-threonine$^8$]cyclosporin B,
[Phosphono-D-threonine$^8$]cyclosporin B disodium salt,
[Phosphono-D-threonine$^8$]cyclosporin B dipotassium salt,
[Phosphono-D-threonine$^8$]cyclosporin C,
[Phosphono-D-threonine$^8$]cyclosporin C disodium salt,
[Phosphono-D-threonine$^8$]cyclosporin C dipotassium salt,
[Phosphono-D-threonine$^8$]cyclosporin D,
[Phosphono-D-threonine$^8$]cyclosporin D disodium salt,
[Phosphono-D-threonine$^8$]cyclosporin D dipotassium salt,
[Phosphono-D-threonine$^8$]cyclosporin E,
[Phosphono-D-threonine$^8$]cyclosporin E disodium salt,
[Phosphono-D-threonine$^8$]cyclosporin E dipotassium salt,
[Phosphono-D-threonine$^8$]cyclosporin F,
[Phosphono-D-threonine$^8$]cyclosporin F disodium salt,
[Phosphono-D-threonine$^8$]cyclosporin F dipotassium salt,
[Phosphono-D-threonine$^8$]cyclosporin G,
[Phosphono-D-threonine$^8$]cyclosporin G disodium salt,
[Phosphono-D-threonine$^8$]cyclosporin G dipotassium salt,
[Phosphono-D-threonine$^8$]cyclosporin H,
[Phosphono-D-threonine$^8$]cyclosporin H disodium salt,
[Phosphono-D-threonine$^8$]cyclosporin H dipotassium salt,
[Phosphono-D-threonine$^8$]cyclosporin I,
[Phosphono-D-threonine$^8$]cyclosporin I disodium salt,
[Phosphono-D-threonine$^8$]cyclosporin I dipotassium salt,
[Phosphono-D-threonine$^8$]cyclosporin K,
[Phosphono-D-threonine$^8$]cyclosporin K disodium salt,
[Phosphono-D-threonine$^8$]cyclosporin K dipotassium salt,
[Phosphono-D-threonine$^8$]cyclosporin L,
[Phosphono-D-threonine$^8$]cyclosporin L disodium salt,
[Phosphono-D-threonine$^8$]cyclosporin L dipotassium salt,
[Phosphono-D-threonine$^8$]cyclosporin M,
[Phosphono-D-threonine$^8$]cyclosporin M disodium salt,
[Phosphono-D-threonine$^8$]cyclosporin M dipotassium salt,
[Phosphono-D-threonine$^8$]cyclosporin O,
[Phosphono-D-threonine$^8$]cyclosporin O disodium salt,
[Phosphono-D-threonine$^8$]cyclosporin O dipotassium salt,

[Phosphono-D-threonine$^{8}$]cyclosporin P,
[Phosphono-D-threonine$^{8}$]cyclosporin P disodium salt,
[Phosphono-D-threonine$^{8}$]cyclosporin P dipotassium salt,
[Phosphono-D-threonine$^{8}$]cyclosporin Q,
[Phosphono-D-threonine$^{8}$]cyclosporin Q disodium salt,
[Phosphono-D-threonine$^{8}$]cyclosporin Q dipotassium salt,
[Phosphono-D-threonine$^{8}$]cyclosporin R,
[Phosphono-D-threonine$^{8}$]cyclosporin R disodium salt,
[Phosphono-D-threonine$^{8}$]cyclosporin R dipotassium salt,
[Phosphono-D-threonine$^{8}$]cyclosporin S,
[Phosphono-D-threonine$^{8}$]cyclosporin S disodium salt,
[Phosphono-D-threonine$^{8}$]cyclosporin S dipotassium salt,
[Phosphono-D-threonine$^{8}$]cyclosporin T,
[Phosphono-D-threonine$^{8}$]cyclosporin T disodium salt,
[Phosphono-D-threonine$^{8}$]cyclosporin T dipotassium salt,
[Phosphono-D-threonine$^{8}$]cyclosporin U,
[Phosphono-D-threonine$^{8}$]cyclosporin U disodium salt,
[Phosphono-D-threonine$^{8}$]cyclosporin U dipotassium salt,
[Phosphono-D-threonine$^{8}$]cyclosporin V,
[Phosphono-D-threonine$^{8}$]cyclosporin V disodium salt,
[Phosphono-D-threonine$^{8}$]cyclosporin V dipotassium salt,
[Phosphono-D-threonine$^{8}$]cyclosporin W,
[Phosphono-D-threonine$^{8}$]cyclosporin W disodium salt,
[Phosphono-D-threonine$^{8}$]cyclosporin W dipotassium salt,
[Phosphono-D-threonine$^{8}$]cyclosporin X,
[Phosphono-D-threonine$^{8}$]cyclosporin X disodium salt,
[Phosphono-D-threonine$^{8}$]cyclosporin X dipotassium salt,
[Phosphono-D-threonine$^{8}$]cyclosporin Y,
[Phosphono-D-threonine$^{8}$]cyclosporin Y disodium salt,
[Phosphono-D-threonine$^{8}$]cyclosporin Y dipotassium salt,
[Phosphono-D-threonine$^{8}$]cyclosporin Z,
[Phosphono-D-threonine$^{8}$]cyclosporin Z disodium salt,
[Phosphono-D-threonine$^{8}$]cyclosporin Z dipotassium salt,
[Phosphono-D-threonine$^{8}$, phosphonothreonine$^{2}$]cyclosporin A,
[Phosphono-D-threonine$^{8}$, phosphonothreonine$^{2}$]cyclosporin A disodium salt,
[Phosphono-D-threonine$^{8}$, phosphonothreonine$^{2}$]cyclosporin A dipotassium salt,
[Phosphonohydroxynorvaline$^{2}$]cyclosporin A,
[Phosphonohydroxynorvaline$^{2}$]cyclosporin A disodium,
[Phosphonohydroxynorvaline$^{2}$]cyclosporin A dipotassium salt,
[Phosphonohydroxynorvaline$^{2}$, phosphono-D-serine$^{8}$]cyclosporin A,
[Phosphonohydroxynorvaline$^{2}$, phosphono-D-serine$^{8}$]cyclosporin A disodium,
[Phosphonohydroxynorvaline$^{2}$, phosphono-D-serine$^{8}$]cyclosporin A dipotassium salt.

Among those compounds, [phosphono-D-serine$^{8}$]cyclosporin A disodium salt is particularly preferred.

## BEST MODE FOR CARRYING OUT THE INVENTION

The technology for producing the compound of the invention is now described.

Various cyclosporins which can be used as starting compounds for the production of the compound of the invention can be synthesized by the known processes. The literature describing such processes include but is not limited to Japanese Kokai Tokkyo Koho S52-59180, Kokai Tokkyo Koho S56-128725, Kokai Tokkyo Koho S57-140753, Kokai Tokkyo Koho S60-215700, Kokai Tokkyo Koho S61-212599, Kokai Tokkyo Koho S62-77399, Kokai Tokkyo Koho H2-137, as well as the literature referred to hereinbefore.

The phosphorylation reaction can also be carried out by the per se known methods. Thus, classified by the kind of phosphorylating agent, there can be mentioned the method which comprises using an acid chloride type phosphorylating agent (e.g. phosphorus oxychloride, phenylphosphoric dichloride, diphenylphosphoryl chloride, dibenzylphosphoryl chloride, p-nitrophenylphosphoric dichloride, dimorpholinophos phoryl chloride, bis(β, β, β-trichloroethyl) phosphate, p-diphenyl-p'-morpholinopyrophosphoryl chloride, etc.), the method which comprises an acid anhydride type phosphorylating agent (e.g. O-benzylphosphorous acid-O-diphenylphosphoric acid mixed anhydride, tetra(p-nitrophenyl)-pyrophosphate, tetrachloropyrophosphate, diphenylphosphoric anhydride, etc.), the method using an imidoylphosphoric acid as an intermediate (β-cyanoethylphosphoric acid plus dicyclohexylcarbodiimide, monobenzylphosphoric acid plus dicyclohexylcarbodiimide, etc.), and the method which comprises using other kinds of phosphorylating agents such as phosphorus trichloride, tris(8-guinolyl) phosphate, 2-(N,N-dimethylamino)-4-nitrophenylphosphoric acid, etc. The solvent that can be used includes pyridine, dimethylformamide, dimethyl sulfoxide, chloroform, methylene chloride, methanol, ethanol, and so forth. When a solvent other than pyridine is used, a suitable base such as pyridine, 2,6-dimethylpyridine, or the like may be added. The reaction temperature is usually 0-100°C but where necessary the reaction can be conduced under further cooling or heating. The reaction time is usually several hours to a few days. In order to obtain the compound of the invention, after phosphorylation the protective group is removed by a method according to the phosphorylating agent used. This removal can also be effected by the known method such as acid or alkaline hydrolysis or catalytic reduction.

For recovery, purification and isolation from the reaction mixture, the known methods applicable to cyclosporins can be utilized [Japanese Kokai Tokkyo Koho S55-55150, Kokai Tokkyo Koho S57-140753, Kokai Tokkyo Koho S62-77399, etc.). Furthermore, as far as the compound of the present invention is concerned, methods utilizing the phosphate group can also be used with advantage. For example, extraction with a solvent such as ethyl acetate, butyl acetate, chloroform, methylene chloride, or n-butanol at a low pH level and subsequent transfer from the solvent layer to an aqueous layer at a high pH level; adsorption on activated carbon, Amberlite XAD (Rohm & Haas Co.), Diaion HP-20 (Mitsubishi Kasei) or the like and subsequent elution with methanol/water or acetone/water; adsorption and elution using an ion exchange resin such as Dowex 1x2 (Dow Chemical), QAE-Sephadex A-25 (Pharmacia), DEAE-Cellulose Whatman DE-32 (Whatman), or DEAE-Sephadex A-25 (Pharmacia); gel filtration using Sephadex G-10 (Pharmacia) or Bio-Gel P-20 (Bio-Rad); column chromatography on cellulose or Avicel SF (American Viscose); forced precipitation by addition of acetone or the like; and freeze-drying, can be carried out each independently, in combination, or in repetition.

The compound of the present invention may exist as the salt of its phosphate group and such a salt can be prepared by the known technology, for example by adding a hydroxide such as sodium hydroxide to the free compound of the invention and stirring the mixture. The salt includes but is not limited to salts with alkali metals such as sodium, potassium, lithium, etc.; salts with alkaline earth metals such as calcium, magnesium, etc.; salts with metals such as aluminum; ammonium salt; and salts with organic bases, e.g. salts with primary, secondary, or tertiary amines such as monoethylamine, dimethylamine, trimethylamine, monoethanolamine, diethanolamine, etc.; and salts with such other organic bases as benzathine, procaine, benzylamine, dibenzylamine, N-benzyl-β-phenethylamine, L-ephenamine, N,N'-dibenzylethylenediamine, dehydroabietylamine, N-ethylpiperidine, dicyclohexylamine, and so forth. Among them, physiologically acceptable salts are preferred and the sodium salt and potassium salt are particularly preferred.

Generally speaking, the compound of the invention is more highly water-soluble in the salt form than in the free form. Therefore, in using the compound in medicinal applications, the salt form is preferably selected according to the intended use.

The compound of the invention may also form solvates inclusive of hydrates. The hydrates form chiefly in the course of synthesis and purification of the compound of the invention and can be isolated as they are. The solvates may be the solvates of the free compound of the invention or the solvates of the salt of the invention.

The compound of the invention may occur as polymorphs. Such polymorphs are also subsumed in the concept of the compound of the invention.

Being the phosphoric ester of the existing cyclosporin, the compound of the invention essentially has the pharmacological efficacies possessed by the existing corresponding cyclosporin. The compound of the invention is particularly useful as a therapeutic drug for immunodeficiency and various diseases associated with immunodeficiency.

Specifically, for example, the compound of the invention is useful for inhibition of graft rejection in kidney transplantation, graft rejection in bone marrow transplantation, prevention of the graft-vs.-host disease, and inhibition of graft rejection in hepatic transplantation, or as a therapeutic drug for psoriasis vulgaris, pustular psoriasis, erythroderma psoriaticum, psoriasis arthropica, etc., diseases of the eye such as Behcet's syndrome (in cases presenting with ocular symptoms), endogenous uveitis, compications of corneal transplantation, vernal keratoconjunctivitis, ligneous conjunctivitis, dry eye, anterior uveitis, onchocercasis, etc.; prevention of rejection of grafts in cardiac, pulmonary, pancreatic, skin and corneal transplantations, therapeutic drugs for various autoimmune diseases such as rheumatoid arthritis, diabetes mellitus I, systemic lupus erythematosus, xeroderma, Wegner's granulomatosis, eosinophilic fasciitis, primary cirrhosis, Graves' disease, Crohn's disease, etc., myasthenia gravis, multiple sclerosis, and other diseases. It is also useful for the treatment of AIDS and AIDS-related diseases, atopic dermatitis, allergic contact dermatitis, and other diseases. Furthermore, the compound is of use as a therapeutic drug for various diseases basically arising from immunodeficiency, such as nephrotic syndrome (frequently recurrent, steroid-resistant), aplastic anemia, and pure red cell aplasia.

For use as a medicine, the compound of the invention can be administered either as it is or in the form of a pharmaceutical composition containing, for example, 0.01-99.5%, preferably 0.5-90%, of the compound and the remainder of a pharmaceutically acceptable nontoxic, inert carrier.

The pharmaceutical composition containing the compound of the invention (hereinafter referred to as the composition of the invention) is now explained.

As the carrier, one or more of solid, semisolid, or liquid diluent, filler, and other formulation auxiliaries can be employed. The pharmaceutical composition is preferably administered in unit dosage forms. The pharmaceutical composition of the invention can be administered orally, parenterally, topically (e.g. transdermally, by instillation into the eye, transnasally, etc.), or rectally. Of course, dosage forms suited for respective routes of administration should be selected. Preferred is oral or parenteral administration, oral administration being particularly preferred. That the oral route of administration is preferred is one of the characteristics of the invention.

The dosage of the compound of the invention as an immunosuppressant is preferably established taking the patient factors, e.g. age, body weight, etc., route of administration, nature and severity of illness, etc. into consideration. Usually, however, the daily dosage for human adults may range generally from 5 mg to 1 g/patient and preferably from 10 to 100 mg/patient. Lower dose levels may be sufficient in some cases, while higher doses may be necessary in other cases. The above-mentioned dosage can be administered in 2-3 divided doses where necessary.

Oral administration can be carried out using solid or liquid unit dosage forms such as bulc powders, capsules, tablets, dragees, granules, powders, suspensions, solutions, syrups, drops, sublingual tablets, and so forth.

Bulk powders can be provided by comminuting the active compound into a finely divided form. Powders can be manufactured by comminuting the active substance into a finely-divided form in the same manner and blending it with a similarly comminuted pharmaceutical carrier, e.g. an edible carbohydrate such as starch, mannitol, or the like. Where necessary, a corrigent, a preservative, a dispersant, a coloring agent, a perfume, etc. can also be added.

Capsules can be manufactured by filling said finely-divided bulk powders, powders, or granules described below for tablets, in capsule shells such as gelatin capsule shells. Preceding the filling operation, a lubricant or a fluidizing agent, such as colloidal silica, talc, magnesium stearate, calcium stearate, solid polyethylene glycol, etc., can be blended with the powders. Improvement in the efficacy of the drug after ingestion can be expected when a disintegrator or a solubilizer, such as carboxymethylcellulose, carboxymethylcellulose calcium, low-substitution-degree hydroxypropylcellulose, croscarmellose sodium, carboxymethylstarch sodium, calcium carbonate, sodium carbonate, etc., is added.

Soft capsules can be provided by suspending said finely divided powders in vegetable oil, polyethylene glycol, glycerin, or a surfactant and accommodating the suspension in gelatin sheets. Tablets can be manufactured by adding an excipient to said powders, granulating or slugging the mixture, adding a disintegrator and/or a lubricant, and compressing the whole mixture. A powdery mixture can be prepared by mixing said finely divided powders with said diluent or base. Where necessary, a binder (e.g. carboxymethylcellulose sodium, methylcellulose, hydroxypropylmethylcellulose, gelatin, polyvinylpyrrolidone, polyvinylalcohol, etc.), a dissolution retardant (e.g. paraffin), a reabsorption promoter (e.g. quaternary salts), and an adsorbent (e.g. bentonite, kaolin, dicalcium phosphate, etc.) can be added. The powdery mixture can be processed into granules by wetting it with a binder, e.g. a syrup, a starch paste, gum arabic, a solution of cellulose, or a solution of a polymer, stirring the mixture, drying it, and pulverizing the same. Instead of granulating such powders, it is possible to compress the powders with a tablet machine and crush the resulting slugs of crude form.

The resulting granules can be protected against caking by the addition of a lubricant such as stearic acid, a salt of stearic acid, talc, mineral oil, or the like. The mixture thus lubricated is then compressed. The resulting core tablets can be coated with a film coating composition or a sugar coating composition.

The compound of the invention can be mixed with a free-flowing inert carrier and the mixture be directly compressed without resort to the above-mentioned granulation or slugging process. A transparent or translucent protective coat consisting in, for example, a hermetic shellac coat, a sugar or polymer coat, or a glazing wax coat can also be applied.

Other oral compositions such as a solution, a syrup, a troche, and an elixir can also be provided in unit dosage forms each containing a predetermined amount of the drug substance. Syrups can be manufactured by dissolving the compound in suitable flavored aqueous media, while elixirs can be manufactured using nontoxic alcoholic vehicles. Suspensions can be provided by dispersing the compound in nontoxic vehicles. Where necessary, solubilizers and emulsifiers (e.g. ethoxylated isostearyl alcohol, polyoxyethylene sorbitol ester, etc.), preservatives, and flavorants (e.g. peppermint oil, saccharin, etc.) can also be added.

Where necessary, the unit dosage formulation for oral administration can be microencapsulated. This formulation can be coated or embedded in a polymer, wax or other matrix to provide a prolonged action or sustained release dosage form.

Parenteral administration can be made using liquid unit dosage forms for subcutaneous, intramuscular, or intravenous injection, e.g. solutions and suspensions. Such unit dosage forms can be manufactured by suspending or dissolving a predetermined amount of the compound of the invention in an injectable nontoxic liquid vehicle, for example an aqueous vehicle or an oily vehicle, and sterilizing the resulting suspension or solution. For isotonizing an injection, a nontoxic salt or salt solution can be added. Moreover, stabilizers, preservatives, emulsifiers, etc. may also be added.

Rectal administration can be carried out by using suppositories manufactured by dissolving or suspending the compound of the invention in a low-melting water-soluble or water-insoluble solid carrier such as polyethylene glycol, caccao butter, semisynthetic oil (e.g. Witepsol, trademark), a higher ester (e.g. myristyl palmitate) or a mixture thereof.

Topical administration can be made using such dosage forms as an ointment, a poultice, a plaster, a patch, a liniment, eye-drops, nasal drops, and so forth. Ointments can be manufactured by admixing the compound of the invention with a fat, fatty oil, lanolin, white petrolatum, paraffin, wax, a resin or plastic, a glycol, a higher alcohol, glycerin, water, or the like together with an emulsifier, suspending agent, and/or other suitable additives. Poultices can be manufactured by mixing a bulk powder of the compound of the invention with a suitable liquid substance such as glycerin, water, or the like and adding an essential oil. Plasters can be manufactured by mixing the compound of the invention evenly with a fat, fatty oil, fatty acid salt, wax, a resin or plastic, purified lanolin, rubber, or the like, or with a base prepared from them. Eye-drops can be manufactured by dissolving or suspending a predetermined amount of the compound of the invention in sterilized pure water, physiological saline, or distilled water for injection and distributing aliquots into applicator bottles.

EXAMPLES

The following working and test examples illustrate the present invention in further detail. The compounds described in the examples are only a few representative species of the compound of the invention.

Example 1 Synthesis of [phosphonothreonine$^2$]cyclosporin A

In 6 ml of water containing 5 ml of a strongly acidic cation exchange resin (AG50W-X2, BIO-RAD) was suspended 484 mg of barium 2-cyanoethylphosphate dihydrate and the suspension was stirred until the insoluble salt had disappeared. The resulting solution was applied onto a column packed with 2 ml of the same resin as above and elution was carried out with 20 ml of water. The eluate was concentrated to dryness under reduced pressure to give cyanoethyl phosphate. The cyanoethyl phosphate used in the subsequent examples was also prepared by the above procedure. To this cyanoethyl phosphate, 187 mg of cyclosporin C, 640 mg of dicyclohexylcarbodiimide, and 10 ml of pyridine were added and the mixture was stirred at room temperature for 63.5 hours. After completion of the reaction, the reaction mixture was diluted with 3 ml of water, stirred for 30 minutes, and filtered through a filter paper. The filtrate was concentrated under reduced pressure and the residue was suspended in 5 ml of methanol and purified by column chromatography (column: 27x850 mm, stationary phase: Sephadex LH20 (Pharmacia), eluent: methanol) to recover 235 mg of a crude fraction. To a 91 mg portion of the above fraction, 8 ml of 25% aqueous ammonia was added and the mixture was stirred at room temperature for 24 hours. The mixture was then adjusted to pH 1-2 with 5N-hydrochloric acid and adsorbed on a reversed phase $C_{18}$ resin (MEGA BOND ELUT $C_{18}$ Cartridge, Analytichem). The resin was rinsed with water and elution was carried out with 60 → 70% methanol. The solvent was then distilled off under reduced pressure to provide 29 mg of white powders. The powders were further purified by preparative high performance liquid chromatography under the following conditions, and from the active fraction the solvent was distilled off under reduced pressure. /Preparative HPLC conditions

Column: Inertsil PREP-ODS, 20x250 mm (G. L. Science)
Column temperature: 60°C
Eluent: 70% acetonitrile-0.3% phosphoric acid/$H_2O$
Flow rate: 7 ml/min.
Retention time of product: 17.2 min.

This fraction was suspended in a small amount of water and adsorbed on a reversed phase $C_{18}$ resin (Sep-Pak $C_{18}$ Cartridge, Waters). After aqueous rinse, elution was carried out with methanol to provide 16 mg of [phosphonothreonine$^2$]cyclosporin A as white powders.

/Physicochemical information on [phosphonothreonine$^2$]cyclosporin A

1. Description of the substance: white powders
2. Melting point: 171-175°C
3. Optical rotation: $[\alpha]^{20}$=-138° (c=0.69, MeOH)
4.

| Elemental analysis, for $C_{62}H_{112}N_{11}O_{16}P \cdot 5/2\ H_2O$ | | | |
|---|---|---|---|
| Calcd. (%): | C, 55.42; | H, 8.78; | N, 11.47 |
| Found (%): | C, 55.14; | H, 8.45; | N, 11.37 |

5. Molecular weight 1297 (FAB MS)
6. Mass spectra:

Anion FAB MS : $(M-H)^-$, m/z 1296
Cation FAB MS: $(M+H)^+$, m/z 1298

Example 2 Synthesis of [phosphonothreonine$^2$]cyclosporin A disodium

To 250 mg of cyclosporin C, 154 mg of cyanoethyl phosphate, 422 mg of dicyclohexylcarbodiimide, and 8 ml of pyridine were added and the mixture was stirred at room temperature for 16 hours. After completion of the reaction, the reaction mixture was diluted with 2 ml of water, stirred for 60 minutes, and filtered to remove insoluble matter. The filtrate was concentrated under reduced pressure, 40 ml of 25% aqueous ammonia was added to the residue, and the mixture was stirred at room temperature for 19 hours. This mixture was concentrated to about 15 ml under reduced pressure, adjusted to pH 1-2 with 2N-hydrochloric acid, and extracted 3 times with 15 ml each of ethyl acetate. The ethyl acetate layer was washed with water, dried over anhydrous sodium sulfate, and concentrated under reduced pressure. The residue was dissolved in 10 ml of methanol, and 0.9 ml of 0.5N-sodium hydroxide was added dropwise. This mixture was stirred at room temperature for 16 hours, after which the solvent was distilled off under reduced pressure. The residue was diluted with 3 ml of water and adsorbed on a reversed phase $C_{18}$ resin (MEGA BOND ELUT $C_{18}$ Cartridge, Analytichem). After the resin was rinsed with water, eluation was carried out with 40 $\rightarrow$ 60% methanol. From the eluate, the solvent was distilled off under reduced pressure to provide 204 mg of the objective disodium salt.

/Physicochemical information on [phosphonothreonine$^2$]cyclosporin A disodium salt

1. Description of the substance: Colorless crystalline powders
2. Melting point: 209-210°C
3. Optical rotation: $[\alpha]^{20}$=-158 ° (c=0.48, MeOH)
4.

| Elemental analysis, for $C_{62}H_{110}N_{11}O_{16}PNa_2 \cdot 3H_2O$ | | | |
|---|---|---|---|
| Calcd. (%): | C, 53.32; | H, 8.37; | N, 11.03 |
| Found (%): | C, 53.03; | H, 8.44; | N, 11.02 |

5. Molecular weight: 1341 (FAB MS)
6. Mass spectrum:

Cation FAB MS: $(M+H)^+$, m/z 1342

Separately, 200 mg of cyclosporin C was phosphorylated in the same manner as above and the cyanoethyl group was cleaved off with 0.2N-sodium hydroxide/$H_2O$. Then, desalting was carried out using Microacylizer G1 and Acyplex Cartridge AC-210-10 (both from Asahi Kasei Co.) and, after concentration under reduced pressure, the residue was purified by column chromatography (column packing: Sephadex LH20 (Pharmacia); eluent: methanol) to provide 16 mg of the product compound as white powders. Based on the determination of molecular mass by FAB MS, this compound was identified to be [phosphonothreonine$^2$]cyclosporin A monosodium salt (Mass spectrum: Cation FAB MS: $(M+H)^+$, m/z 1320).

Example 3 Synthesis of [phosphono-D-serine[8]]cyclosporin A

To 0.7 g of [D-serine[8]]cyclosporin A, 0.5 g of cyanoethyl phosphate, 1.5 g of dicyclohexylcarbodiimide, and 20 ml of pyridine were added and the mixture was stirred for 20 hours. The reaction mixture was diluted with 5 ml of water, stirred for 1 hour, and filtered through a filter paper to remove insoluble matter. The filtrate was concentrated to dryness under reduced pressure, 100 ml of aqueous ammonia was added to the residue, and the mixture was stirred at room temperature overnight. This mixture was then adjusted to pH 1-2 with hydrochloric acid and extracted 3 times with 150 ml each of ethyl acetate. The extract was dehydrated over anhydrous sodium sulfate and the solvent was distilled off under reduced pressure. The residue was purified by column chromatography (column: 27x270 mm, stationary phase: Sephadex LH20 (Pharmacia); eluent: methanol) to provide 0.34 g of [phosphono-D-serine[8]]cyclosporin A.

/Physicochemical information on [phosphono-D-serine[8]]cyclosporin A

1. Description of the substance: white powders
2. Melting point: 170-174°C
3. Optical rotation: $[\alpha]^{20}$=-167 ° (c=0.51, MeOH)
4.

| Elemental analysis, for $C_{62}H_{112}N_{11}O_{16}P \cdot 3/2H_2O$ | | | |
|---|---|---|---|
| Calcd. (%): | C, 56.18; | H, 8.74; | N, 11.62 |
| Found (%): | C, 56.12; | H, 8.55; | N, 11.45 |

5. Molecular weight: 1297 (FAB MS)
6. Mass spectra:

Anion FAB MS : $(M-H)^-$, m/z 1296
Cation FAB MS: $(M+H)^+$, m/z 1298

Example 4 Synthesis of [phosphono-D-serine[8]]cyclosporin A disodium

[D-serine[8]]cyclosporin A was phosphorylated as in Example 3. To 120 mg of [D-serine[8]]cyclosporin A, 74 mg of cyanoethyl phosphate, 204 mg of dicyclohexylcarbodiimide, and 4 ml of pyridine were added and the mixture was stirred for 64 hours. The reaction mixture was then diluted with 0.5 ml of water, stirred for 30 minutes, and filtered through a filter paper to remove insoluble matter. The filtrate was concentrated to dryness under reduced pressure, 20 ml of 25% aqueous ammonia was added to the residue, and the mixture was stirred for 21 hours and then filtered through a filter paper again to remove insoluble matter. The filtrate was concentrated to dryness under reduced pressure. Then, 0.7 ml of 0.5N sodium hydroxide/$H_2O$ was added and the mixture was stirred for 20 hours. The solvent was then distilled off under reduced pressure and the residue was adsorbed on a reversed phase $C_{18}$ resin (MEGA BOND ELUT $C_{18}$ Cartridge, Analytichem). After the resin was rinsed with water, elution was carried out with 40% methanol. From the eluate, the solvent was distilled off under reduced pressure to provide 70 mg of the objective disodium salt.

/Physicochemical information on [phosphono-D-serine[8]]cyclosporin A disodium salt

1. Description of the substance: Colorless crystalline powders
2. Melting point: 199-203°C
3. Optical rotation: $[\alpha]^{20}$=-159 ° (c=0.27, MeOH)
4.

| Elemental analysis, for $C_{62}H_{110}N_{11}O_{16}PNa_2 \cdot 5H_2O$ | | | |
|---|---|---|---|
| Calcd. (%): | C, 51.98; | H, 8.44; | N, 10.75 |
| Found (%): | C, 51.72; | H, 8.15; | N, 10.74 |

5. Molecular weight: 1341 (FAB MS)
6. Mass spectrum:

Cation FAB MS: $(M+H)^+$, m/z 1342

Example 5 Synthesis of [phosphono-D-serine$^8$]cyclosporin A dibenzylamine salt

In 1 ml of water was dissolved 20 mg of the [phosphono-D-serine$^8$]cyclosporin A disodium salt obtained in Example 4. This solution was adjusted to pH 1-2 with 2N-hydrochloric acid and extracted 3 times with 1 ml each of ethyl acetate. The extract was dried over anhydrous sodium sulfate and concentrated to dryness under reduced pressure to give 17 mg of a white substance. To this substance, 0.5 ml of methanol and 4 µl of benzylamine were added in that order and the mixture was stirred overnight. The solvent was then distilled off under reduced pressure and the residue was diluted with 1 ml of water and adsorbed on a reversed phase $C_{18}$ resin (SEP-PAK $C_{18}$ Cartridge, Waters). After the resin was rinsed with water, elution was carried out with 40% methanol. From the eluate, the solvent was distilled off under reduced pressure to provide 13 mg of the objective dibenzylamine salt.

/Physicochemical information on [phosphono-D-serine$^8$]cyclosporin A dibenzylamine salt

1. Description of the substance: white powders
2. Melting point: 164-166°C
3. Optical rotation: $[\alpha]^{20}$=-199 ° (c=0.28, MeOH)
4.

| Elemental analysis, for $C_{76}H_{130}N_{13}O_{16}P \cdot 7H_2O$ | | | |
|---|---|---|---|
| Calcd. (%): | C, 55.69; | H, 8.86; | N, 11.11 |
| Found (%): | C, 55.98; | H, 8.55; | N, 11.17 |

Example 6 Synthesis of [phosphonothreonine$^2$, phosphono-D-serine$^8$]cyclosporin A tetrasodium salt

[D-serine$^8$]cyclosporin C was phosphorylated as in Example 3. To 30 mg of [D-serine$^8$]cyclosporin C, 39 mg of cyanoethyl phosphate, 102 mg of dicyclohexylcarbodiimide, and 2 ml of pyridine were added and the mixture was stirred for 65 hours. This reaction mixture was diluted with 0.5 ml of water, further stirred for 30 minutes, and filtered through a filter paper to remove insoluble matter. The filtrate was concentrated to dryness under reduced pressure, 6 ml of aqueous ammonia was added to the residue, and the mixture was stirred for 21 hours and then filtered through a filter paper again to remove insoluble matter. The filtrate was concentrated to dryness under reduced pressure, 0.2 ml of 0.5N-sodium hydroxide/$H_2O$ was added to the residue, and the mixture was stirred for 20 hours. The solvent was then distilled off and the residue was adsorbed on a reversed phase $C_{18}$ resin (SEP-PAK $C_{18}$ Cartridge, Waters). After the resin was rinsed with water, elution was carried out with 40% methanol. From the eluate, the solvent was distilled off under reduced pressure to provide 8 mg of the objective tetrasodium salt as white powders. Based on the determination of molecular mass by FAB MS, this compound was identified to be [phosphonothreonine$^2$, phosphono-D-serine$^8$]cyclosporin A tetrasodium salt (mass spectrum: Cation FAB MS: $(M+H)^+$, m/z 1482).

Test Example 1 IL-2 production inhibitory activity

It is known that the immunosuppressant action of cyclosporins is based on inhibition of the IL-2 gene of T cells. Therefore, the biological activity of cyclosporins can be assayed using inhibition of the IL-2 production of T cells as an indicator. Accordingly, using Jurkat cells (a tumor cell line derived from human T cells), the biological activity of the compound of the invention was evaluated.

Jurkat cells grown to the logarithmic phase in RPMI-1640 medium (Flow Laboratories; 10% heat-inactivated FBS, 300 mg/l L-glutamine, 50 U/ml streptomycin, 50 U/ml penicillin, 25 mM Hepes, 50 µM 2-mercaptoethanol) were seeded onto a 96-well microtiter plate at a density of 8x105 cells/ml, 0.1 ml/well. Then, as an IL-2 production inducer, 10 µl of concanavalin A was added, followed by addition of 20 µl each of samples varying in concentration. After the final volume was adjusted to 0.2 ml/well with the medium, the plate was incubated in a $CO_2$ incubator at 37°C for 24 hours. After completion of culture, the reaction mixture in each well was centrifuged (1000 rpm x 5 min.) and the supernatant was recovered.

Determination of IL-2 was carried out by the bioassay using CTLL-2 cells which are known as IL-2-dependent cells.

To 0.1 ml of CTLL-2 cells (40,000/ml) in logarithmic phase, 0.1 ml of an appropriate dilution of the above supernatant was added, and the cells were cultured in a $CO_2$ incubator at 37°C for 48 hours. After completion of culture, the cell growth was determined by the MTT assay. The relation between growth of CTLL-2 cells and IL-2 concentration was previously plotted for a recombinant IL-2 and using the plots as a standard curve, the IL-2 concentration was estimated from the degree of growth of CTLL-2 cells.

The IL-2 production inhibition rate was calculated by means of the following equation (1) and the $IC_{50}$ value of each sample was calculated from the inhibition rates at various concentrations. The results are shown in Table 2.

$$\text{IL-2 production inhibition rate (\%)} = \left(1- \frac{\text{IL-2 production by cells in presence of sample}}{\text{IL-2 production by cells in absence of sample}}\right) \times 100 \qquad (1)$$

Table 2

| Sample identification | IL-2 production inhibitory concentration ($IC_{50}$ ng/ml) |
|---|---|
| Cyclosporin A | 3 |
| Cyclosporin C | 12 |
| [D-serine$^8$]cyclosporin A | 6 |
| [Phosphonothreonine$^2$]cyclosporin A | 49 |
| [Phosphono-D-serine$^8$]cyclosporin A | 19 |

It will be apparent from Table 2 that the compound of the invention is not so active as the non-phosphorylated compound and yet has potent IL-2 production inhibitory activity of its own.

Test Example 2 <u>Solubility in water</u>

Cyclosporin A, cyclosporin C, [D-serine$^8$]cyclosporin A, [phosphono-D-serine$^8$]cyclospoin A disodium salt, and [phosphonothreonine$^2$]cyclosporin A disodium salt were respectively ground in a mortar and aliquots were put in test tubes. After distilled water was added to each tube to make the concentration of the sample 250 mg/ml, the tubes were shaken at 30 °C for 30 minutes. From each test tube, 250 µl was taken into a centrifuge tube and centrifuged (10,000 rpm x 10 min.). The supernatant was recovered, diluted with distilled water, and subjected to HPLC for assay.

As to [phosphono-D-serine$^8$]cyclosporin A disodium salt and [phosphonothreonine$^2$]cyclosporin A disodium salt, a sufficient amount of distilled water was added to make 500 mg/ml and otherwise the above procedure was repeated to determine the solubility again.

HPLC conditions

| | |
|---|---|
| Column: | Inertsil C8 (4.6x250 mm) |
| Mobile phase: | acetonitrile-methanol-distilled water-phosphoric acid (50:35:15:0.005) |
| Column temperature: | 75°C |
| Detection: | UV 210 nm |

Table 3

| Sample identification | Solubility (mg/ml) |
|---|---|
| Cyclosporin A | 0.0267 |
| Cyclosporin C | 0.0297 |
| [D-serine$^8$]cyclosporin A | 0.0351 |
| [Phosphono-D-serine$^8$]cyclosporin A disodium salt | 500< |
| [Phosphonothreonine$^2$]cyclosporin A disodium salt | 250< |

Table 3 shows the results in contrast to the results for known cyclosporins A and C. It will be apparent from Table 3 that compared with known cyclosporins A and C, the solubility of the compound of the invention in water was not less than about 7000 times as high.

Test Example 3 Nephrotoxicity study

Nephrotoxicity was investigated by the lithium clearance method. The usefulness of lithium clearance as an indicator of renal disorder is well documented (Takashi Takei, Japanese Journal of Urology, 83, 40-47, 1992).

Male 7-week-old SD rats were assigned, 10 animals each, to 5 groups, i.e. a control group, a cyclosporin A 10 mg/kg group, a cyclosporin C group, a [phosphonothreonine$^2$]cyclosporin A group, and a [phosphono-D-serine$^8$]cyclosporin A group. Each sample was adjusted to a concentration of 5 mg/ml and 2 ml/kg was administered intraperitonally for 7 consecutive days. The control group similarly received the solvent used for preparation of the sample solutions. On the last day of administration, an aqueous solution of lithium carbonate (23.6 mg/10 ml/kg) was administered by oral gavage and, then, the animals were put on a lithium diet ($LiCO_3$ 738.9 mg/kg food) prepared by adding lithium carbonate to F-2 rodent food (Funabashi Farm). On the following day the blood was drawn from the jugular vein and the animals were placed in metabolism cages. Under feeding with the lithium diet, the 24-hour urine was collected and the urine volume was measured using a measuring cylinder. After the rats were taken out from the metabolism cages, blood sampling was immediately carried out and the mean lithium value of two measurements before and after urine collection was taken as the blood lithium value of the corresponding rat. The body weight of rats was determined immediately after they were taken out from metabolism cages. To determine the blood lithium concentration, each blood sample was centrifuged at 3000 rpm for 10 minutes and the plasma was taken for determination. The urinary lithium concentration was determined by centrifuging each urine sample at 1500 rpm for 10 minutes and quantitating the lithium in the supernatant with a flame photometer (Model 775, Hitachi, Ltd.). From the measured values, the 24-hour lithium clearance ($C_{Li}$) value per 100 g rat body weight was calculated by means of the following equation (2).

$$24\text{-hr } C_{Li}/100\text{ g} = \text{urine volume} \times 100/\text{rat body weight} \cdot \text{urinary Li value/blood Li value} \qquad (2)$$

Table 4

| Sample identification | (%)* |
|---|---|
| Control | 100 |
| Cyclosporin A | 53 |
| Cyclosporin C | 39 |
| [Phosphonothreonine$^2$]cyclosporin A disodium salt | 125 |
| [Phosphono-D-serine$^8$]cyclosporin A disodium salt | 98 |

* The percentage of blood lithium in each test group relative to control group

Table 4 shows the results in contrast to cyclosporins A and C. In the table, any figure smaller than 100 for control signifies nephrotoxicity. It will be apparent from Table 4 that whereas the known cyclosporins A and C are nephrotoxic, the nephrotoxicity of the compound of the invention is very low.

Test Example 4 Absorption after oral administration and release of the phosphate group

Using SD rats weighing 250 g, [D-serine$^8$]cyclosporin A and [phosphono-D-serine$^8$]cyclosporin A, each suspended in a 0.5% aqueous solution of methylcellulose, were respectively administered orally in a dose of 30 mg/kg and the blood was serially drawn from the jugular vein. Each blood sample was centrifuged (10,000 rpm x 5 min.) and the supernatant was applied onto a pretreatment column (Sep-Pack $C_{18}$ Vac, Waters). After the column was rinsed with distilled water and 40% methanol, elution was carried out in 10% steps from 50% methanol to 100% methanol and 50 $\mu$l of the eluate was injected into an HPLC. From the concentration value thus found, the blood concentration was calculated. The results are shown in Table 5.

Table 5

| Time (hr) after administration | 0.5 | 1 | 2 | 4 | 8 | 24 | Sum of blood concentrations up to 24 hr |
|---|---|---|---|---|---|---|---|
| (1) | 895 | 4321 | 1915 | 1021 | 533 | 69 | 8754 |
| (2) | 1413 | 3115 | 2516 | 2391 | 910 | 103 | 10448 |
| (3) | 391 | 813 | 971 | 536 | 311 | 31 | 3053 |
| (4) | 1804 | 3928 | 3437 | 2927 | 1221 | 134 | 13501 |

In the table, each blood concentration value is in ng/ml.
(1) [D-serine$^8$]cyclosporin A
(2) Blood concentration of [D-serine$^8$]cyclosporin A which released phosphoric acid after administration of [phosphono-D-serine$^8$]cyclosporin A
(3) Blood concentration of [phosphono-D-serine$^8$]cyclosporin A after administration of [phosphono-D-serine$^8$]cyclosporin A
(4) Sum of (2) and (3)

It will be apparent from Table 5 that compared with known cyclosporins (unphosphorylated compounds), not only the absorption rate was higher but also the total amount of adsorption was about 1.5-fold greater. It can also be seen in Table 5 that the compound of the invention released phosphoric acid in vivo and became the non-phosphorylated compound having more potent biological activity.

FORMULATION EXAMPLES

Formulation Example 1 Solid dosage form

Tablets were prepared according to the following recipe per tablet (180 mg).

| Recipe | Compound of Example 4 | 100 mg |
|---|---|---|
| | Lactose | 61 mg |
| | Starch | 14 mg |
| | Polyvinyl alcohol | 2 mg |
| | Talc | 2 mg |
| | Magnesium stearate | 1 mg |

Formulation Example 2 Injection

An injection was prepared according to the following recipe per ampule (1 ml).

| Recipe | Compound of Example 4 | 10 mg |
|---|---|---|
| | Sodium chloride | 90 mg |
| | Sodium hydrogencarbonate | 10 mg |
| | Distilled water for injection | q.s. |

Formulation Example 3 Capsules

Hard capsules were prepared according to the following recipe per capsule (285 mg).

| | | |
|---|---|---|
| Recipe | Compound of Example 4 | 100 mg |
| | Lactose | 107 mg |
| | Microcrystalline cellulose | 10 mg |
| | Magnesium stearate | 3 mg |

EFFECT OF THE INVENTION

The compound of the present invention has potent biological activity of its own and is partially transformed into the non-phosphorylated compound having more potent activity in vivo. The compound is highly soluble in water and shows a high absorption rate and an increased amount of absorption. Therefore, it can be administered without being processed into special pharmaceutical forms and be expected to express its efficacy in vivo.

Furthermore, the nephrotoxicity of the compound of the invention is extremely low. It remains to be further elucidated why the nephrotoxicity of the compound of the invention is so low despite its partial hydrolysis in vivo to the non-phosphorylated compound.

## Claims

**1.** A cyclosporin phosphate derivative which is available upon total or partial phosphorylation [$-P(O)(OH)_2$] of hydroxyl groups of a cyclosporin having hydroxyl group-containing amino acids as its constituent amino acids or a salt thereof, or a solvate thereof.

**2.** The cyclosporin phosphate derivative, salt, or solvate according to Claim 1 wherein said cyclosporin having hydroxyl group-containing amino acids is a compound of the following chemical formula.

$$\underset{1}{MeBmt}-\underset{2}{A_1}-\underset{3}{Sar}-\underset{4}{A_2}-\underset{5}{A_3}-\underset{6}{A_4}-\underset{7}{A_5}-\underset{8}{A_6}-\underset{9}{A_7}-\underset{10}{A_8}-\underset{11}{A_9} \quad [I]$$

wherein $A_1$ represents D- or L- aminobutyric acid, D- or L-alanine, D- or L-threonine, D- or L-valine, D- or L-3-hydroxynorvaline, or D- or L-4-hydroxynorvaline; $A_2$ represents D- or L-N-methylleucine, D- or L-leucine, or D- or L-valine; $A_3$ represents D- or L-valine or D- or L-norvaline; $A_4$ represents D- or L-leucine or D- or L-methylleucine; $A_5$ represents D- or L-alanine or D- or L-$\alpha$-aminobutyric acid; $A_6$ represents D- or L-alanine or D- or L-serine; $A_7$ represents D- or L-methylleucine or D- or L-leucine; $A_8$ represents D- or L-methylleucine or D- or L-leucine, $A_9$ represents D- or L-valine or D- or L-methylvaline; MeBmt represents N-methyl-(4R)-4-but-2E-en-1-yl-4-methyl-(L) threonine of the following chemical formula [II] or the corresponding dihydro compound of the following chemical formula [III].

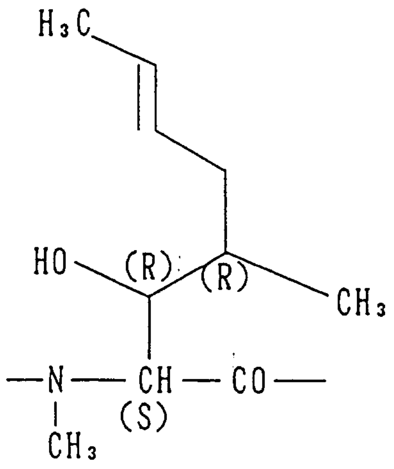

(II)

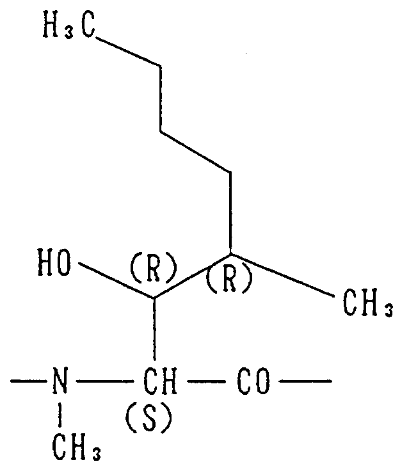

(III)

3. The cyclosporin phosphate derivative, salt, or solvate according to Claim 2 wherein $A_1$ is L-threonine.

4. The cyclosporin phosphate derivative, salt, or solvate according to Claim 2 wherein $A_6$ is D-serine.

5. A cyclosporin phosphate derivative selected from the group consisting of [phosphonothreonine$^2$]cyclosporin A, [phosphono-4-hydroxynorvaline$^2$]cyclosporin A, [phosphono-D-serine$^8$]cyclosporin A, and [phosphonothreonine$^2$, phosphono-D-serine$^8$]cyclosporin A or a salt thereof, or a solvate thereof.

6. A pharmaceutical composition comprising a cyclosporin phosphate derivative, salt, or solvate as claimed in Claims 1 through 5.

7. An immunosuppressant comprising a cyclosporin phosphate derivative, salt, or solvate as claimed in Claims 1 through 5.

## INTERNATIONAL SEARCH REPORT

International application No.
PCT/JP95/01906

**A. CLASSIFICATION OF SUBJECT MATTER**

Int. $Cl^6$ C07K7/64, A61K38/13

According to International Patent Classification (IPC) or to both national classification and IPC

**B. FIELDS SEARCHED**

Minimum documentation searched (classification system followed by classification symbols)

Int. $Cl^6$ C07K7/64, A61K38/13

Documentation searched other than minimum documentation to the extent that such documents are included in the fields searched

Electronic data base consulted during the international search (name of data base and, where practicable, search terms used)

CAS ONLINE

**C. DOCUMENTS CONSIDERED TO BE RELEVANT**

| Category* | Citation of document, with indication, where appropriate, of the relevant passages | Relevant to claim No. |
|---|---|---|
| A | JP, 5-508653, A (Vacsyn France, S.A.), December 2, 1993 (02. 12. 93), Claims 1, 15 WO, 92/01475, A1 & EP, 539457, A1 | 1 - 7 |
| A | JP, 3-218396, A (Sandoz AG.), September 25, 1991 (25. 09. 91), Claim & EP, 414632, A2 & CA, 2021788, A & US, 5284826, A | 1 - 7 |

☐ Further documents are listed in the continuation of Box C. ☐ See patent family annex.

* Special categories of cited documents:
- "A" document defining the general state of the art which is not considered to be of particular relevance
- "E" earlier document but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| December 12, 1995 (12. 12. 95) | January 16, 1996 (16. 01. 96) |

| Name and mailing address of the ISA/ | Authorized officer |
|---|---|
| Japanese Patent Office | |
| Facsimile No. | Telephone No. |

Form PCT/ISA/210 (second sheet) (July 1992)